(19) Europäisches Patentamt
European Patent Office
Office européen des brevets

(11) Veröffentlichungsnummer: **0 074 561**
**B1**

# (12) EUROPÄISCHE PATENTSCHRIFT

(45) Veröffentlichungstag der Patentschrift: **21.11.84**

(21) Anmeldenummer: **82108028.0**

(22) Anmeldetag: **01.09.82**

(51) Int. Cl.³: **C 07 C 49/523**, C 07 C 49/757, C 07 C 45/49, C 07 C 119/045, C 07 C 87/36 // C08G18/75, C09D3/72

## (54) Cyclische Keto-butyraldehyde, ein Verfahren zu ihrer Herstellung und ihre Verwendung zur Herstellung von cyclischen Diisocyanaten.

(30) Priorität: **10.09.81 DE 3135948**

(43) Veröffentlichungstag der Anmeldung: **23.03.83 Patentblatt 83/12**

(45) Bekanntmachung des Hinweises auf die Patenterteilung: **21.11.84 Patentblatt 84/47**

(84) Benannte Vertragsstaaten: **BE CH DE FR GB IT LI NL**

(56) Entgegenhaltungen: **PATENTS ABSTRACTS OF JAPAN, Band 3, Nr. 50(C-44), 27. April 1979, Seite 29C44**

(73) Patentinhaber: **BAYER AG, Konzernverwaltung RP Patentabteilung, D-5090 Leverkusen 1 Bayerwerk (DE)**

(72) Erfinder: **Klein, Gerhard, Dr., von-Flotow-Strasse 7, D-4019 Monheim (DE)**
Erfinder: **Arlt, Dieter, Dr. Prof., Rybniker Strasse 2, D-5000 Köln 80 (DE)**
Erfinder: **Braden, Rudolf, Dr., Nothauser Feld 1, D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 074 561 B1

BUNDESDRUCKEREI BERLIN

**Beschreibung**

Die Erfindung betrifft neue cyclische Keto-butyraldehyde und ihre Herstellung aus cyclischen Ketoolefinen. Die neuen cyclischen Keto-butyraldehyde können für die Herstellung von cyclischen Diisocyanaten verwendet werden, die als Vernetzer in Polymeren eingesetzt werden können.

Endprodukte, im besonderen in der Polymerchemie, basieren in der Regel auf Ausgangsstoffen, die aus dem Erdöl stammen. Bei der allgemeinen Verknappung der Erdölprodukte besteht bei der Entwicklung neuer Produkte ein Bedürfnis von Ausgangsstoffen auszugehen, die unabhängig vom Erdöl sind und sich immer wieder neu bilden.

Gegenstand der vorliegenden Erfindung sind neue cyclische Keto-butyraldehyde, die sich von Kohlenwasserstoffen vom Limonen-Typ ableiten.

Die neuen cyclischen Keto-butyraldehyde entsprechen der Formel (I)

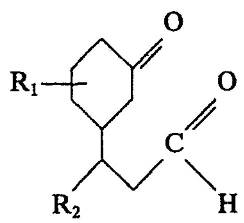

(I)

in der

$R_1$ und $R_2$ entweder gleich oder verschieden sind und Wasserstoff oder Niederalkyl bedeuten und in der der cycloaliphatische Ring gegebenenfalls eine Doppelbindung enthält.

Niederalkyl kann im Rahmen der vorliegenden Erfindung ein geradkettiger oder verzweigter Kohlenwasserstoffrest mit 1 bis etwa 6 Kohlenstoffatomen sein. Beispielsweise seien Methyl, Ethyl, Propyl, Isopropyl, Butyl, Isobutyl, Pentyl, Isopentyl, Hexyl und Isohexyl genannt.

Cyclische Keto-butyraldehyde der Formel (II)

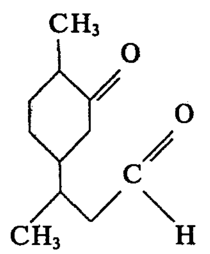

(II)

in der

der cycloaliphatische Ring gegebenenfalls eine Doppelbindung enthält,

werden im allgemeinen bevorzugt.

Im einzelnen seien die folgenden cyclischen Keto-butyraldehyde genannt:

3-[4-Methyl-3-oxo-cyclohexyl]-butyraldehyd,
3-[4-Methyl-3-oxo-cyclohex-4-enyl]-butyraldehyd,
3-[3-Oxo-cyclohexyl]-propionaldehyd und
3-[4-Methyl-3-oxo-cyclohexyl]-propionaldehyd.

Die erfindungsgemäßen neuen cyclischen Keto-butyraldehyde können als Riechstoffe verwendet werden. Im besonderen ist es aber möglich, sie zu den entsprechenden Diaminen und diese zu den Diisocyanaten umzusetzen. Die cyclischen Diisocyanate können vorteilhaft als Vernetzer in der Polymerchemie, insbesondere bei Polyamiden und Polyurethanen, verwendet werden.

Die neuen cyclischen Keto-butyraldehyde können hergestellt werden, wenn man cyclische Ketoolefine der Formel (III)

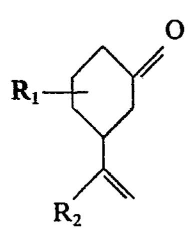

(III)

in der

$R_1$ und $R_2$ entweder gleich oder verschieden und Wasserstoff oder Niederalkyl bedeuten und in der der

cycloaliphatische Ring gegebenenfalls eine Doppelbindung enthält
in Gegenwart eines Rhodium-haltigen Katalysators mit Kohlenmonoxid und Wasserstoff im Temperaturbereich von 50 bis 200° C und bei einem Druck von mindestens 30 bar umsetzt.

Cyclische Keto-olefine für das erfindungsgemäße Verfahren, wie das Carvon und das Dihydrocarvon, können aus Limonen hergestellt werden. Limonen ist Bestandteil von Citrusfrüchten, wie Organgen, Zitronen und Limonen, und wird im allgemeinen durch eine Destillation der Fruchtschalen gewonnen.

Die Herstellung von Carvon aus Limonen ist bekannt. So kann z. B. durch Addition von Nitrosylchlorid an die endocyclische Doppelbindung des Limonens eine dimere Chlornitrosoverbindung erhalten werden. Diese läßt sich mit Basen in das Carvonoxim überführen, das sauer zu Carvon hydrolysiert werden kann (Ind. Eng. Chem., 43, 1196 (1951)). Diese dreistufige Umsetzung läßt sich auch als sogenanntes Eintopfverfahren durchführen (US-PS 3 293 301).

Die Darstellung des Dihydrocarvons ist ebenfalls bekannt. Beispielsweise läßt sich Limonen mit Persäuren zu Limonenmonoepoxid umsetzen (J. Amer. Chem. Soc., 77, 3405 (1955)). Dieses Epoxid kann mit Lewis-Säuren (Helv. Chim. Acta 47, 413 (1964)) oder mit sauren Ionenaustauschern (Za. Vses. Khem. Oesaca. 22, 223 (1977)) zu Dihydrocarvon umgelagert werden.

Die Herstellung der cyclischen Keto-olefine kann auch mit Hilfe einer Diels-Alder-Reaktion erfolgen. In diesem Fall setzt man ein geeignetes Dien mit Isopren gegebenenfalls in Gegenwart einer Lewis-Säure um.

Die Hydroformylierung der cyclischen Keto-olefine mit Kohlenmonoxid/Wasserstoff nach dem erfindungsgemäßen Verfahren wird in Gegenwart eines Rhodium-Katalysators durchgeführt. Die Hydroformylierung von Limonen ist an sich bekannt (New Synthesis with Carbon Monoxide, Springer Verlag Berlin (1980), Seiten 109—119). Es war aber nicht zu erwarten, daß sich das bekannte Verfahren auf die gegebenenfalls ungesättigten cyclischen Keto-olefine übertragen läßt, da sie neben zwei Doppelbindungen zusätzlich noch durch eine Ketogruppe substituiert sind, die unter den Reaktionsbedingungen bevorzugt reagieren sollte.

Bevorzugte Rhodiumkomplexe für das erfindungsgemäße Verfahren enthalten einen oder mehrere Stickstoff-, Phosphor- und/oder Schwefel-haltige Liganden.

Besonders bevorzugte, als Katalysatoren verwendete Rhodiumkomplexe entsprechen den Formeln

$XRh(CO)L_2$ $HRh(CO)L_3$, $HRh(CO)_2L_2$, $RhXL_3$, $[Rh(CO)_2L_2]$, $[Rh(OCOCH_3)(CO)L]_2$,

wobei X für ein Chlor-, Brom oder Jodatom und L für einen organischen Liganden steht. Geeignete organische Liganden können tertiäre organische Phosphine, Sulfide, Sulfone und tertiäre Amine sein. Geeignete Liganden sind beispielsweise: Tertiäre organische Phosphine, die als organische Reste jeweils höchstens bis zu zwei gleiche oder verschiedene Alkylreste mit 1 bis 20 Kohlenstoffatomen, Cycloalkylreste mit 5 bis 12 Kohlenstoffatomen, Aralkylreste mit 7 bis 10 Kohlenstoffatomen und mindestens einen Arylrest mit 6 bis 10 Kohlenstoffatomen haben. Die genannten Reste können unter Reaktionsbedingungen inerte Substituenten haben, z. B. 1 bis 2 Hydroxylgruppen, Alkoxy- oder Carbalkoxygruppen mit 1 bis 4 Kohlenstoffatomen, Aminogruppen oder Halogenatome, wie Triphenylphosphin, Diethylphenylphosphin, Tritolylphosphin, Trinaphthylphosphin, Diphenylmethylphosphin, Diphenylbutylphosphin, Tris-(p-chlorphenyl)-phosphin, Tris-(p-carbmethoxyphenyl)-phosphin, Tris-(p-cyanophenyl)-phosphin, Diphenyl-phosphonigsäurephenylester, Benzol-phosphonigsäure-di-phenyl-ester und Diphenyl-(dimethylamino)-phenylphosphin.

$P[CH_2CH_2CH_2N(CH_3)_2]_3$

$P[CH_2CH_2CH_2N(C_2H_5)_2]_3$

$P(CH_2CH_2CH_2-\underset{H}{N}-iso\text{-}C_4H_9)_3$

$P[CH_2CH_2CH_2N(iso\text{-}C_4H_9)_2]_3$

$(n\text{-}C_4H_9)_2PCH_2CH_2N(C_2H_5)_2$

$P[CH_2N(C_2H_5)_2]_3$

$P[C_6H_4N(CH_3)_2]_3$

$P[CH_2CH_2C_6H_4N(C_2H_5)_2]_3$

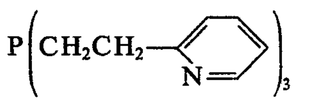

$P[CH_2CH_2CH_2N(tert.C_4H_9)_2]_3$ und

$P[CH_2CH_2CH_2N(iso\text{-}C_3H_7)_2]_3$

Die verwendeten phosphorhaltigen Liganden werden am zweckmäßigsten aus der Gruppe der Phosphine ausgewählt. Besonders bevorzugte Liganden sind Triphenylphosphin und Tributylphosphin.

Ebenfalls gemäß der Erfindung verwendbar sind komplexe Liganden in Form von teilweise durch Ferrocen substituierte Triorganophosphine (DE-OS 2 617 306). Es ist jedoch jeder Triorganophosphorligand, der für rhodiumkatalysierte Hydroformylierungs-Reaktionssysteme geeignet ist, verwendbar.

Geeignete stickstoffhaltige Liganden sind beispielsweise: Pyridin, Picoline, Ethylpyridine, N-Methylpyrrolidin, N-Methylpyrrol, N,N'-Dimethylpiperazin, Dimethylcyclohexylamin, Triethylamin, N,N-Dimethylanilin, N-Methylmorphidin, N-Methylindol, Chinolin, Isochinolin, N-Methylpyrrolidon und 3-Dimethylaminopropionitril.

Geeignete schwefelhaltige Liganden sind beispielsweise: Dibenzylsulfid, Di-n-butylsulfid, Dimethylsulfoxid, Diethylsulfid, Di-(4-Chlor-benzyl)-sulfid, Di-(4-Cyanobenzyl)-sulfid, Bis-(4-dimethylaminobenzyl)-sulfid, Di-(4-diethylaminobenzyl)-sulfid, Di-($\alpha$-naphthylmethyl)-sulfid, Di-(2,6-dichlorbenzyl)-sulfid, Di-(3,4-dichlorbenzyl)-sulfid, Di-(2-chlorbenzyl)-sulfid, Di-(5,6,7,8-tetrahydronaphthyl-2-methyl)-sulfid, Benzyl-methyl-sulfid, Benzyl-dodecyl-sulfid, 4-Dimethylaminobenzyl-methyl-sulfid, Benzyl-butyl-sulfid, Bis-(4-carboxybenzyl)-sulfid, Di-(4-methyl-benzyl)-sulfid, Di-(3-methylbenzyl)-sulfid, Di-(-methylbenzyl)-sulfid und Tetramethylensulfon.

Im Katalysatorkomplex kann das Mengenverhältnis Rhodium zu Ligand in einem weiten Bereich verändert werden. Im allgemeinen enthält das Reaktionsmedium jedoch mindestens 1 Mol des Liganden pro Grammatom Rhodium. Der Ligand kann aber auch in hohem Überschuß zugesetzt werden, so daß ein Molverhältnis von Ligand zu Rhodium von 1 bis zu 200 : 1 eingesetzt wird. Bevorzugt verwendet man jedoch ein Molverhältnis Ligand zu Rhodium von 10 bis 80 : 1. Es kann auch vorteilhaft sein, die Ligandenverbindung als Lösungsmittel zu verwenden.

In einer bevorzugten Ausführungsform des erfindungsgemäßen Verfahrens präformiert man den aktiven Katalysator in einem Lösungsmittel bei erhöhter Temperatur und erhöhtem Druck, z. B. einem Wassergas-Druck von 200 bar, und gibt unter den Reaktionsbedingungen das cyclische Keto-olefin hinzu.

Pro kg des cyclischen Keto-olefins setzt man erfindungsgemäß 1 bis 1000 mg Rhodiummetall im Katalysator ein. Im besonderen werden 10 bis 600 mg Rhodiummetall pro kg des cyclischen Keto-olefins verwendet. Der Katalysator kann nach bekannten Methoden wiedergewonnen und erneut verwendet werden (DE-OS 1 954 315 (1969) und DE-OS 2 311 388 (1973)).

Bei der erfindungsgemäßen Hydroformylierung werden Kohlenmonoxid und Wasserstoff im allgemeinen mindestens im stöchiometrischen Verhältnis, vorteilhaft jedoch im Überschuß bis zu 100 Mol%, angewendet. Das Gemisch aus Kohlenmonoxid und Wasserstoff enthält Kohlenmonoxid und Wasserstoff in der Regel im Volumenverhältnis von 1 : 4 bis 4 : 1, insbesondere im Verhältnis 2 : 1 bis 1 : 2.

Die erfindungsgemäße Hydroformylierung führt man im allgemeinen im Temperaturbereich von 50 bis 200°C durch. Besonders bevorzugt werden Temperaturen im Bereich von 90 bis 180°C. Das erfindungsgemäße Verfahren wird im allgemeinen bei einem Druck von wenigstens 30 bar durchgeführt. Bevorzugt führt man das erfindungsgemäße Verfahren im Druckbereich von 70 bis 400 bar durch.

Im allgemeinen wird das erfindungsgemäße Verfahren in einer Zeit von 20 bis 120 Minuten bis zu einem praktisch vollständigen Umsatz durchgeführt.

Das erfindungsgemäße Verfahren wird im allgemeinen in flüssiger Phase durchgeführt. Es ist hierbei vorteilhaft, den Katalysator auf einen festen Träger, z. B. Kieselgel, zu fixieren.

Das flüssige Reaktionsmedium kann entweder ein Gemisch von an sich vorhandenen Flüssigkeiten (Reaktionsprodukten oder überschüssige Ligand-Verbindung) darstellen oder gegebenenfalls auch ein zugesetztes Lösungsmittel enthalten, das unter den Reaktionsbedingungen sich nicht verändert und in dem der Katalysator und der überschüssige Ligand löslich sind. Lösungsmittel sind beispielsweise Hexan, Octan, Cyclohexan, Benzol, Toluol, Xylol.

In einer bevorzugten Ausführungsform des erfindungsgemäßen Verfahrens wird die Hydroformylierung des cyclischen Keto-olefins zu dem erfindungsgemäßen cyclischen Keto-butyraldehyd in Gegenwart eines Hydrido-Carbonyl-Phosphin-Rhodium-Komplexes und einem Überschuß des Phosphin-Liganden, der einem Phosphor- zu Rhodium-Verhältnis von 10 bis 80 : 1 entspricht, in einem Kohlenwasserstoff als Lösungsmittel im Temperaturbereich von 130 bis 165° C und im Druckbereich von 150 bis 350 bar mit Wasserstoff und Kohlenmonoxid im Verhältnis 0,7 bis 1,5 durchgeführt.

Erfindungsgemäß können die neuen cyclischen Keto-butyraldehyde zur Herstellung von Diisocyanaten der Formel (IV)

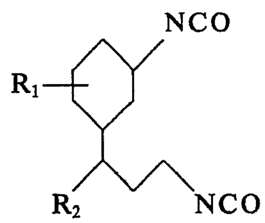

(IV)

in der

$R_1$ und $R_2$ die obengenannte Bedeutung haben, verwendet werden, indem man den cyclischen Ketobutyraldehyd in Gegenwart eines Hydrierkatalysators mit Ammoniak und Wasserstoff im Temperaturbereich von 50 bis 280° C und im Druckbereich von 10 bis 200 bar zu Diaminen der Formel (V)

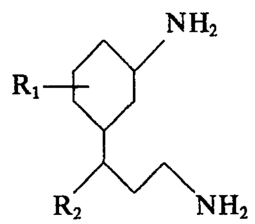

(V)

in der

$R_1$ und $R_2$ die obengenannte Bedeutung haben, umsetzt und dann diese Diamine in einem inerten Lösungsmittel mit einer Säure behandelt und dabei gebildete Salze im Temperaturbereich von 20 bis 250° C mit Phosgen umsetzt.

Die Diisocyanate der Formel (IV) und die Diamine der Formel (V) sind ebenfalls neu.

Die reduktive Aminierung ist an sich bekannt (Houben-Weyl, Methoden der Organischen Chemie, Band 4, Teil 1/C (1980), Seiten 412ff).

Erfindungsgemäß erhält man das Diamin in hoher Selektivität, wenn man bei einem Molverhältnis Ammoniak/cyclisches Keto-butyraldehyd von wenigstens 2,2 : 1, bevorzugt von 5 bis 15 : 1, arbeitet. Die reduktive Aminierung kann diskontinuierlich als Sumpfphasenhydrierung oder kontinuierlich, beispielsweise in der Rieselphase, durchgeführt werden.

Es kann vorteilhaft sein, ein unter den Bedingungen der erfindungsgemäßen Aminierung inertes Lösungsmittel zu verwenden. Als Lösungsmittel seien beispielsweise Alkohole, Ether, Amide oder heterocyclische Verbindungen wie Methanol, Ethanol, Isopropanol, Butanol, Ethylenglykol, Ethylenglykoldimethylether, Tetrahydrofuran, N-Methylpyrrolidon, Dimethylformamid und Hexan genannt. Bevorzugt im Lösungsmittel sind Ethanol und Isopropanol.

Im allgemeinen wird die erfindungsgemäße reduktive Aminierung im Temperaturbereich von 50 bis 180°C, bevorzugt von 90 bis 135°C, durchgeführt. Der Wasserstoffdruck sollte größer als 10 bar sein und im Bereich von 50 bis 200 bar liegen.

Es ist vorteilhaft, die erfindungsgemäße Aminierung unter Zusatz eines Ammoniumsalzes wie Ammoniumacetat durchzuführen. In diesem Fall setzt man im allgemeinen 0,1 bis 5% des Ammoniumsalzes, bezogen auf den Ketoaldehyd ein.

Hydrierkatalysatoren für die erfindungsgemäße reduktive Aminierung enthalten im allgemeinen als aktive Komponenten mindestens eines der Metalle Vanadium, Chrom, Mangan, Eisen, Kobalt, Nickel oder Kupfer in reduzierter und/oder oxidischer Form. Bevorzugte Katalysatoren sind Nickel- oder Kobalt-Katalysatoren, in Form von Trägerkatalysatoren, wobei als Träger anorganische Materialien wie Kieselgur, Kieselsäure, Aluminiumoxide, Silikate, Aluminiumsilikate, Montmorillonite, Zeolithe, Spinelle, Dolomit, Kaolin, Magnesiumsilikate, Zirkonoxid, Eisenoxid, Zinkoxid, Calciumcarbonat, Siliciumcarbid, Aluminiumphosphat, Borphosphat, Asbest oder Aktivkohle und als organische Katalysatorträger natürlich vorkommende oder synthetische Verbindungen mit hohem Molekulargewicht wie Seide, Polyamide, Polystyrole, Zellstoff oder Polyurethane verwendbar sind, wobei die Träger in Form von Kuchen, Strängen, Fäden, Zylindern Polygonen oder in Pulverform vorliegen können.

Bevorzugte Katalysatoren für die erfindungsgemäße Aminierung sind auch Katalysatoren vom Raney-Typ, wie Raney-Nickel, W-1-, W-5-, W-6-, W-7-Raney-Nickel (J. Am. Chem. Soc. 69, 3039 (1974)), Raney-Kobalt-Katalysatoren, Raney-Kupfer, Raney-Nickel-Eisen, Raney-Kobalt-Nickel, Raney-Kobalt-Eisen, durch Reduktion von Nickel- oder Kobaltsalzen hergestellte Metallkatalysatoren, wie Urushibara-Nickel oder mit Metallalkylverbindungen, Alkalihydriden, Hydrazin, Bornaten oder Borwasserstoff reduzierte Nickel- oder Kobaltsalze oder durch Reduktion der Metalloxide oder Metalloxidgemische hergestellte Katalysatoren.

Die Reduktion der Katalysatoren kann mit Wasserstoff, gegebenenfalls bei erhöhter Temperatur und erhöhtem Druck oder unter den Bedingungen des erfindungsgemäßen Verfahrens oder während des Verfahrens erfolgen.

Die Hydrierkatalysatoren können als Beschleuniger eines oder mehrere der folgenden Elemente in einer Menge bis zu 10 Gew.-% enthalten: Lithium, Natrium, Calcium, Barium, Kalium, Silber, Beryllium, Lanthan, Cer, Titan, Vanadium, Niob, Tantal, Molybdän oder Wolfram. Außerdem können bis zu 1 Gew.-% der Elemente Ruthenium, Rhenium, Palladium, Gold, Iridium und Platin in dem Hydrierkatalysator enthalten sein.

Besonders bevorzugte Hydrierkatalysatoren für die erfindungsgemäße reduktive Aminierung sind Raney-Katalysatoren wie Raney-Nickel, Raney-Kobalt und Raney-Nickel-Eisen.

Das durch reduktive Aminierung hergestellte Diamin wird in an sich bekannter Weise mit Phosgen zu dem cyclischen Diisocyanat umgesetzt. Dazu wird gegebenenfalls in einem inerten Lösungsmittel das Diamin mit einer gasförmigen Säure wie Chlorwasserstoff oder Kohlendioxid zu dem entsprechenden Additionsprodukt umgesetzt und anschließend mit Phosgen behandelt.

Im allgemeinen setzt man 2 bis 30 Mol, bevorzugt 5 bis 10 Mol, Phosgen mit 1 Mol des Diamins um.

Unter Abspaltung der Säure entsteht dabei das cyclische Diisocyanat. Die Reaktionstemperatur liegt im allgemeinen im Bereich von 20 bis 250° C, bevorzugt von 130 bis 170° C.

Für die Phosgenierung können als Lösungsmittel alle üblichen Lösungsmittel verwendet werden, die sich unter den Reaktionsbedingungen nicht verändern und deren Siedepunkt für die Phosgenierung hoch genug liegt und die zum Diisocyanat eine ausreichende Siededifferenz aufweisen. Bevorzugt sind Chlorbenzole, Nitrobenzole, Xylole, Tetralin und Decalin. Das Diisocyanat kann nach dem Abdestillieren des Lösungsmittels in an sich bekannter Weise durch Destillation gereinigt werden.

Die erfindungsgemäßen cyclischen Diisocyanate können entsprechend DE-OS 2 234 507 als Vernetzer bei der Herstellung von Polyurethanen verwendet werden.

## Beispiel 1

In einem Edelstahlautoklaven werden 2000 g Carvon und 5000 ml Toluol in Gegenwart von 1,3 g Rhodium-2-ethylhexanoat und 152 g Triphenylphosphin bei 150° C und 280 bar mit einem 1 : 1-Gemisch von Wasserstoff und Kohlenmonoxid umgesetzt. Nach 2 Stunden erfolgt keine Gasaufnahme mehr. Das Lösungsmittel wird bei 10 mbar abdestilliert. Der Rückstand wird bei 0,1 mbar fraktioniert. Es werden 1910 g (80% bezogen auf eingesetztes Carvon) eines Gemischs erhalten, das zu 80% aus 3-[4-Methyl-3-oxo-cyclohex-4-enyl]-butyraldehyd, zu 15% aus 3-[4-Methyl-3-oxo-cyclohexyl]-butyraldehyd und zu 5% aus einem gesättigten Oxoalkohol besteht. $Kp \cdot_{0,1} = 100-105°C$.

Der Destillationsrückstand enthält den Hydroformylierungskatalysator und kann für eine erneute Hydroformylierung verwendet werden.

## Beispiel 2

Wie in Beispiel 1 beschrieben, werden 50 g Carvon mit 1,15 g Triphenylphosphin und 0,01 mg Tristriphenylphosphin-Rhodiumchlorid in 250 ml Toluol bei 150° C und 280 bar während 4 Stunden mit einem 1 : 1-Gemisch von Wasserstoff und Kohlenmonoxid umgesetzt. Die destillative Aufarbeitung ergibt 49 g (82% bezogen auf eingesetztes Carvon) eines Gemisches ($Kp \cdot_{0,1} = 101-105°C$), das die gleiche Zusammensetzung hat wie in Beispiel 1 beschrieben.

Beispiel 3

Wie in Beispiel 1 beschrieben, werden 50 g Dihydrocarvon, 1,5 g Triphenylphosphin, 0,013 g Rhodium-2-ethylhexanoat in 200 ml Toluol bei 150°C und 280 bar mit einem Wasserstoff-Kohlenmonoxid-1 : 1-Gemisch umgesetzt. Die destillative Aufarbeitung ergibt 51,5 g 3-[4-Methyl-3-oxo-cyclohexyl]-butyraldehyd (Kp · $_{0,1}$ = 98—100° C, 86% der Theorie).

Beispiel 4

In einem Rührautoklaven aus Edelstahl werden 100 g 3-[4-Methyl-3-oxo-cyclohex-4-enyl]-butyraldehyd, 30 g Raney-Nickel, 5 g Ammoniumacetat und 750 ml Ethanol gefüllt. Der Autoklav wird verschlossen, mit Stickstoff gespült, 350 ml Ammoniak werden flüssig eingepumpt. Unter 80 bar Wasserstoffdruck wird auf 100° C erwärmt und mit Wasserstoff der Druck 1 Stunde bei 110 bar gehalten, aus dem abgekühlten Reaktionsgemisch wird nach Abdampfen des Ammoniaks der Katalysator abfiltriert und das Lösungsmmittel abdestilliert. Die Destillation ergibt 76,5 g 3-[3-Amino-4-methyl-cyclohexyl]-butylamin, kp.$_{0,1}$ = 108—111° C (75% der Theorie).

Beispiel 5

100 g 3-[3-Amino-4-methylcyclohexyl]-butylamin werden in 750 ml trockenem Chlorbenzol gelöst und bei 0°C mit Kohlendioxid gesättigt. Die Kühlung wird entfernt, und unter Einleiten von Phosgen wird zum Sieden erhitzt. Bei 150° C leitet man 8 Stunden Phosgen ein. Aus der jetzt klaren Lösung wird mit Stickstoff nichtumgesetztes Phosgen ausgeblasen. Das Lösungsmittel wird bei 10 mbar abdestilliert und der Rückstand über eine 10 cm Vigreuxkolonne destilliert. Man erhält 89,5 g 3-[3-Isocyanato-4-methylcyclohexyl]-butylisocyanat, kp · $_{0,3}$ 125—127° C.

Beispiel 6 (Anwendungsbeispiel)

Es sollte ein Polyurethanlack aus einem Polyol und einem erfindungsgemäßen Diisocyanat hergestellt werden.

Als Polyol wurde ein Polyether verwendet, der durch Umsetzung von Trimethylpropan mit Propylenoxid und unter Alkalikatalyse hergestellt wurde und eine Hydroxy-Zahl von 380 hat. 147,g g des Polyols wurden mit 131,2 g 3-[3-Isocyanato-4-methylcyclohexyl]-butylisocyanat (nach Beispiel 5) unter Zusatz von 0,1 Gew.-% (bezogen auf das Gesamtgewicht) Dibutylzinnlaurat gemischt und auf eine Metallfolie aufgetragen.

Der Lack klebt 8 Stunden nicht mehr (klebfrei). Nach 7 Tagen ist die Lackschicht gegenüber den Lösungsmitteln Toluol, Aceton und Ethylenglykoldiacetat beständig und besitzt eine hohe Elastizität und Kratzfestigkeit.

**Patentansprüche**

1. Cyclische Keto-butyraldehyde der Formel (I)

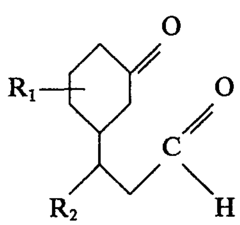

(I)

in der

$R_1$ und $R_2$ entweder gleich oder verschieden sind und Wasserstoff oder Niederalkyl bedeuten und in der der cycloaliphatische Ring gegebenenfalls eine Doppelbindung enthält.

2. Cyclische Keto-butyraldehyde nach Anspruch 1 der Formel (II)

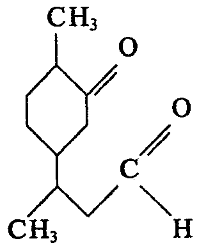

(II)

in der

der cycloaliphatische Ring gegebenenfalls eine Doppelbindung enthält.

3. Verfahren zur Herstellung von cyclischen Keto-butyraldehyden, dadurch gekennzeichnet, daß man cyclische Keto-olefine der Formel (III)

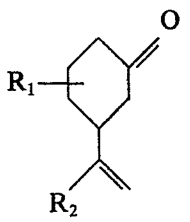

(III)

in der

$R_1$ und $R_2$ entweder gleich oder verschieden sind und Wasserstoff oder Niederalkyl bedeuten und in der der cycloaliphatische Ring gegebenenfalls eine Doppelbindung enthält,

in Gegenwart eines Rhodium-haltigen Katalysators mit Kohlenmonoxid und Wasserstoff im Temperaturbereich von 50 bis 200° C und bei einem Druck von mindestens 30 bar umsetzt.

4. Verfahren nach Anspruch 3, dadurch gekennzeichnet, daß die Umsetzung mit einem Überschuß an Kohlenmonoxid und Wasserstoff durchgeführt wird.

5. Verfahren nach Ansprüchen 3 und 4, dadurch gekennzeichnet, daß als Katalysatoren Rhodium-Komplexe verwendet werden, die einen oder mehrere Stickstoff-, Phosphor- oder Schwefel-haltige Liganden tragen.

6. Verfahren nach den Ansprüchen 3 bis 5, dadurch gekennzeichnet, daß es im Druckbereich von 70 bis 400 bar durchgeführt wird.

7. Verwendung von cyclischen Keto-butyraldehyden nach Anspruch 1 zur Herstellung von Diisocyanaten der Formel (IV)

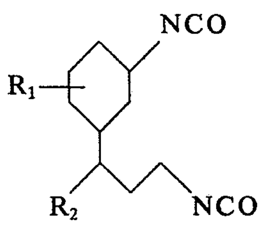

(IV)

in der

$R_1$ und $R_2$ die obengenannte Bedeutung haben,

dadurch gekennzeichnet, daß man den cyclischen Keto-butyraldehyd in Gegenwart eines Hydrierkatalysators mit Ammoniak und Wasserstoff im Temperaturbereich von 50 bis 280° C und im Druckbereich von 10 bis 200 bar zu einem Diamin der Formel (V)

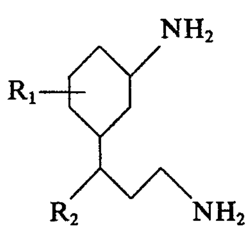

(V)

in der

$R_1$ und $R_2$ die obengenannte Bedeutung haben,

umsetzt und dann dieses Diamin in einem inerten Lösungsmittel mit einer Säure behandelt und das dabei gebildete Salz im Temperaturbereich von 20 bis 250° C mit Phosgen umsetzt.

## Claims

1. Cyclic keto-butyraldehydes of the formula (I)

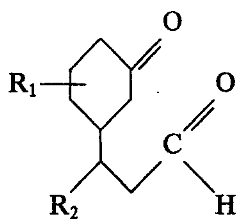

(I)

in which
$R_1$ and $R_2$ are either identical or different and denote hydrogen or lower alkyl and in which the cycloaliphatic ring may contain a double bond.

2. Cyclic keto-butyraldehydes according to Claim 1 of the formula (II)

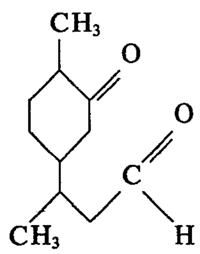

(II)

in which
the cycloaliphatic ring may contain a double bond.

3. Process for the preparation of cyclic keto-butyraldehydes, characterised in that cyclic keto-olefines of the formula (III)

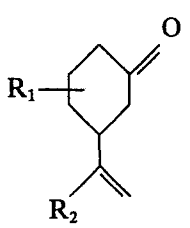

(III)

in which
$R_1$ and $R_2$ are either identical or different and denote hydrogen or lower alkyl and in which the cycloaliphatic ring may contain a double bond,
are reacted in the presence of a rhodium-containing catalyst with carbon monoxide and hydrogen within a temperature range of 50 to 200° C and under a pressure of at least 30 bar.

4. Process according to Claim 3, characterised in that the reaction is carried out with an excess of carbon monoxide and hydrogen.

5. Process according to Claims 3 and 4, characterised in that the catalysts used are rhodium complexes which carry one or more nitrogen-, phosphorus-, or sulphur-containing ligands.

6. Process according to Claims 3 to 5, characterised in that it is carried out within a pressure range of 70 to 400 bar.

7. Use for cyclic keto-butyraldehydes according to Claim 1 for preparing diisocyanates of the formule (IV)

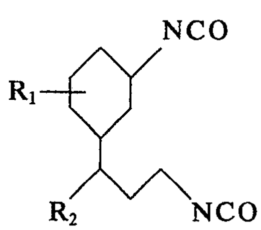

(IV)

in which
$R_1$ and $R_2$ have the abovementioned meaning,
characterised in that the cyclic keto-butyraldehyde is reacted in the presence of a hydrogenation catalyst with ammonia and hydrogen within a temperature range of 50 to 280° C and within a pressure

range of 10 to 200 bar to give a diamine of the formula (V)

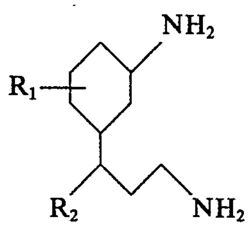

(V)

in which
$R_1$ and $R_2$ have the abovementioned meaning,
and then this diamine is treated in an inert solvent with an acid and the salt thereby formed is reacted within a temperature range of 20 to 250° C with phosgene.

**Revendications**

1. Céto-butyraldéhydes cycliques de formule (I)

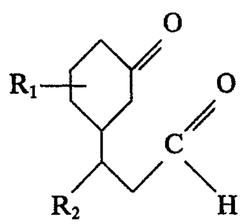

(I)

dans laquelle
$R_1$ et $R_2$ sont égaux ou différents et désignent l'hydrogène ou un groupe alkyle inférieur et le noyau cycloaliphatique comprend éventuellement une double liaison.

2. Céto-butyraldéhydes cycliques suivant la revendication 1, de formule (II)

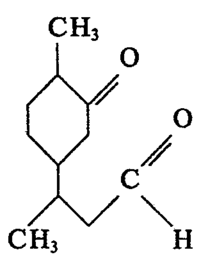

(II)

dans laquelle le noyau cycloaliphatique comporte éventuellement une double liaison.

3. Procédé de production de céto-butyraldéhydes cycliques, caractérisé en ce qu'on fait réagir des céto-oléfines cycliques de formule (III)

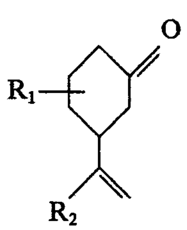

(III)

dans laquelle
$R_1$ et $R_2$ sont égaux ou différents et représentent l'hydrogène ou un groupe alkyle inférieur et le noyau cycloaliphatique comporte éventuellement une double liaison,
en présence d'un catalyseur contenant du rhodium avec l'oxyde de carbone et l'hydrogène dans la plage de températures de 50 à 200° C et à une pression d'au moins 30 bars.

4. Procédé suivant la revendication 3, caractérisé en ce qu'on conduit la réaction avec un excès d'oxyde de carbone et d'hydrogène.

5. Procédé suivant les revendications 3 et 4, caractérisé en ce qu'on utilise comme catalyseurs des complexes de rhodium qui portent un ou plusieurs coordinats contenant de l'azote, du phosphore ou du soufre.

6. Procédé suivant les revendications 3 à 5, caractérisé en ce qu'il est mis en œuvre dans l'intervalle

de pression de 70 à 400 bars.

7. Utilisation de céto-butyraldéhydes cycliques suivant la revendication 1 pour la production de diisocyanates de formule (IV)

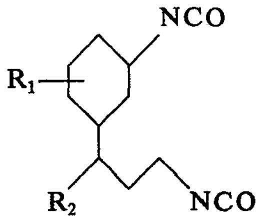

(IV)

dans laquelle
$R_1$ et $R_2$ ont la définition indiquée ci-dessus,
caractérisée en ce qu'on fait réagir le céto-butyraldéhydes cyclique en présence d'un catalyseur d'hydrogénation avec l'ammoniac et l'hydrogène dans l'intervalle de température de 50 à 280°C et dans l'intervalle de pression de 10 à 200 bars pur former une diamine de formule (V)

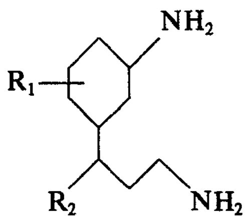

(V)

dans laquelle
$R_1$ et $R_2$ ont la définition indiquée ci-dessus, puis on traite cette diamine dans un solvant inerte avec un acide et on fait réagir le sel ainsi formé avec le phosgène dans l'intervalle de température de 20 à 250°C.